Europäisches Patentamt

European Patent Office

Office européen des brevets

(19)

(11) Publication number: **0 139 383**
A1

## EUROPEAN PATENT APPLICATION

(21) Application number: 84305571.6

(22) Date of filing: 16.08.84

(51) Int. Cl.⁴: **C 12 N 15/00**
C 12 P 21/00, C 12 N 9/99
A 61 K 37/02
//C12R1/85

(30) Priority: 16.08.83 US 523685

(43) Date of publication of application:
02.05.85 Bulletin 85/18

(84) Designated Contracting States:
AT BE CH DE FR GB IT LI LU NL SE

(71) Applicant: ZYMOGENETICS, INC.
2121 North 35th Street
Seattle Washington 98103(US)

(72) Inventor: Russell, Paul R.
6061 26th Avenue, N.E.
Seattle Washington 98115(US)

(74) Representative: Bizley, Richard Edward et al,
BOULT, WADE & TENNANT 27 Furnival Street
London EC4A 1PQ(GB)

(54) Method for expressing foreign genes in schizosaccharomyces pombe and the use in therapeutic formulations of the products, DNA constructs and transformant strains of schizosaccharomyces pombe usable in such method and their preparation.

(57) A method is provided for producing heterologous polypeptides in yeast *Schizosaccharomyces pombe*. DNA constructs are provided for use in this method comprising a segment coding for an *S. pombe* promoter. A particular construct is provided comprising the *S. pombe* alcohol dehydrogenase promoter and human alpha-1-antitrypsin gene, which is used to transform *S. pombe* to produce a substantially unglycosylated protein having activity of human alpha-1-antitrypsin.

METHOD FOR EXPRESSING FOREIGN GENES IN SCHIZOSACCHAROMYCES POMBE AND THE USE IN THERAPEUTIC FORMULATIONS OF THE PRODUCTS, DNA CONSTRUCTS AND TRANSFORMENT STRAINS OF SACCHAROMYCES POMBE USABLE IN SUCH METHOD AND THEIR PREPARATION

The present invention is directed to methods for expressing heterologous polypeptides in the fission yeast Schizosaccharomyces pombe, to DNA vectors useful therein, and to protein products expressed thereby.

In the field of genetic engineering where the object is normally to obtain high expression of a polypeptide product, usually a mammalian protein, in a microorganism, the choice of the host organism may be critical. Some work has been done in the eucaryotic organism Saccharomyces cerevisiae (Baker's yeast). However, whether other eucaryotic microorganisms may achieve similar or even higher levels of expression of heterologous proteins was not heretofore known. The fission yeast Schizosaccharomyces pombe is only distantly related to S. cerevisiae and the two genera differ in many aspects of biochemistry and physiology. Such differences, for example, may be reflected in possible different levels and/or specificities of proteases in the different species which may degrade heterologous proteins. Also, the yeast genera may differ in enzymes which modify heterologous proteins, which may involve differing specific cleavage sites, sulfhydryl bridges, glycosylation, amino-terminal modifications and the like. Depending upon the particular heterologous protein which one may express, such specific modifications may be desirable or undesirable. Furthermore, different yeast genera may contain different proteins which in some cases may be more difficult than others to purify away from the desired heterologous protein. Also, at certain levels the presence of heterologous proteins may have a detrimental effect on cell viability, so it may be desirable to determine which yeast genera may be most tolerant to the particular desired protein. The secretion processes may differ among yeast genera, therefore one may be able to take advantage of such properties, for example, a particular heterologous protein may be secreted at a higher level in one genus versus another, or undesirable proteases and protein modifying enzymes may be secreted at a lower level while the heterologous protein is secreted at a higher level in certain genera. Finally, yeast genera may differ in their growth rates or yields in different media at different temperatures and pressures, thereby affecting time and cost of production. It is therefore desirable to provide a method of producing foreign proteins in different eucaryotic organisms to take advantage of such differences in characteristics. In most cases, such differences in characteristics may not be determined a priori.

It is therefore an aspect of the present invention to provide methods for producing heterologous proteins in the fission yeast Schizosaccharomyces pombe.

It is a further aspect of the present invention to provide DNA vectors which are useful for expressing heterologous proteins in S. pombe.

It is another aspect of the present invention to provide DNA expression vectors comprising S. pombe alcohol dehydrogenase promoter.

It is a further aspect of the present invention to provide substantially pure unglycosylated human alpha-1 antitrypsin produced by Schizosaccharomyces pombe.

In the accompanying FIGURES:

FIG. 1 is the cDNA sequence of the gene coding for the predominant form of human alpha-1 antitrypsin.

FIG. 2 illustrates a partial sequence of pUC13 including the linker.

FIG. 3 illustrates a method of preparation of vector HAT 4.

FIG. 4 illustrates the restriction map of vector C1/1, a vector utilized in preparation of HAT 4.

FIG. 5 illustrates the restriction map of the vector pUC α-1 + FG1, an intermediate vector used in the preparation of HAT 4.

FIG. 6 illustrates the restriction map of HAT 4.

FIG. 7 illustrates a method of preparing vector pR103-5, an expression vector for producing alpha-1 antitrypsin in S. pombe.

FIG. 8 illustrates the restriction map of pR103-5.

The present invention is directed to a method for producing a heterologous protein in yeast genus S. pombe comprising the step of growing a culture of the yeast transformed by a DNA vector containing a segment coding for an S. pombe promoter, whereby expression of the protein is under regulation of the promoter. DNA constructs useful for making such vectors are also provided. In particular, a vector is constructed comprising the S. pombe alcohol dehydrogenase promoter and the structural gene for human alpha-1 antitrypsin which is transformed into S. pombe. Growth of the transformant produces a substantially unglycosylated protein having the activity of mammalian alpha-1 antitrypsin. This protein may be isolated in a substantially pure form.

In order to express heterologous proteins in S. pombe, it is desirable to have the expression under control of a promoter which is indigenous to S. pombe. Such a promoter may be the approximately 700 base pair Sph I-Eco RI fragment comprising the S. pombe ADH promoter which may be isolated from plasmid pADHpoI, a plasmid which is disclosed by Russell et al., J.Biol.Chem., 258, 143-149 (1983), the disclosure of which is incorporated by reference herein in its entirety. Other S. pombe promoters may include the glycolytic pathway promoters, such as, triose phosphate isomerase, phosphofructo kinase, glyceraldrhyde-3-phosphate dehydrogenase. The heterologous protein selected for expression is alpha-1 antitrypsin, a protease inhibitor present in mammalian blood whose apparently major physiological function is to inhibit elastase, a potent protease which hydrolyzes structural proteins. Alpha-1 antitrypsin may also inhibit other serine proteases. A low level of alpha-1 antitrypsin in the blood may be associated with chronic obstructive pulmonary emphysema and infantile liver cirrhosis. Therefore, production of proteins having alpha-1 antitrypsin activity may be useful for treating alpha-1 antitrypsin deficiency and to examine the mechanism of the acute-phase response which occurs under inflamatory conditions in the body whereby the concentration of alpha-1 antitrypsin is substantially increased.

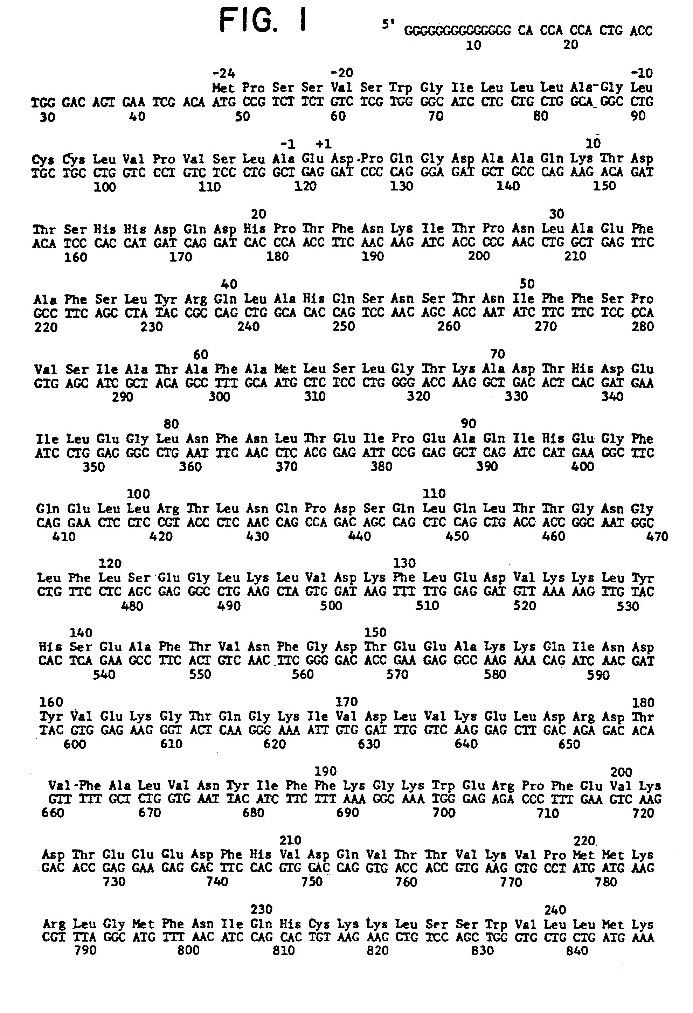

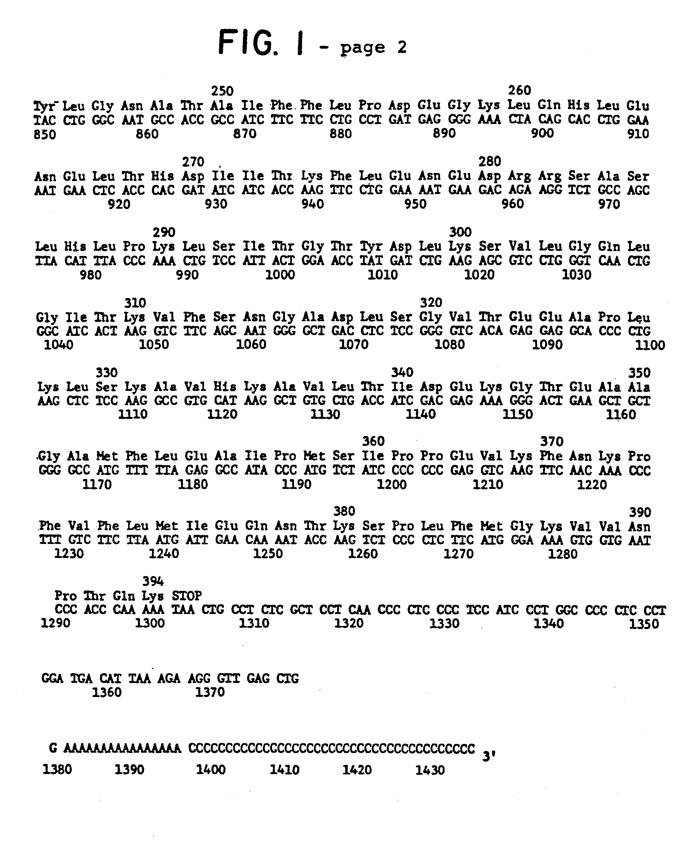

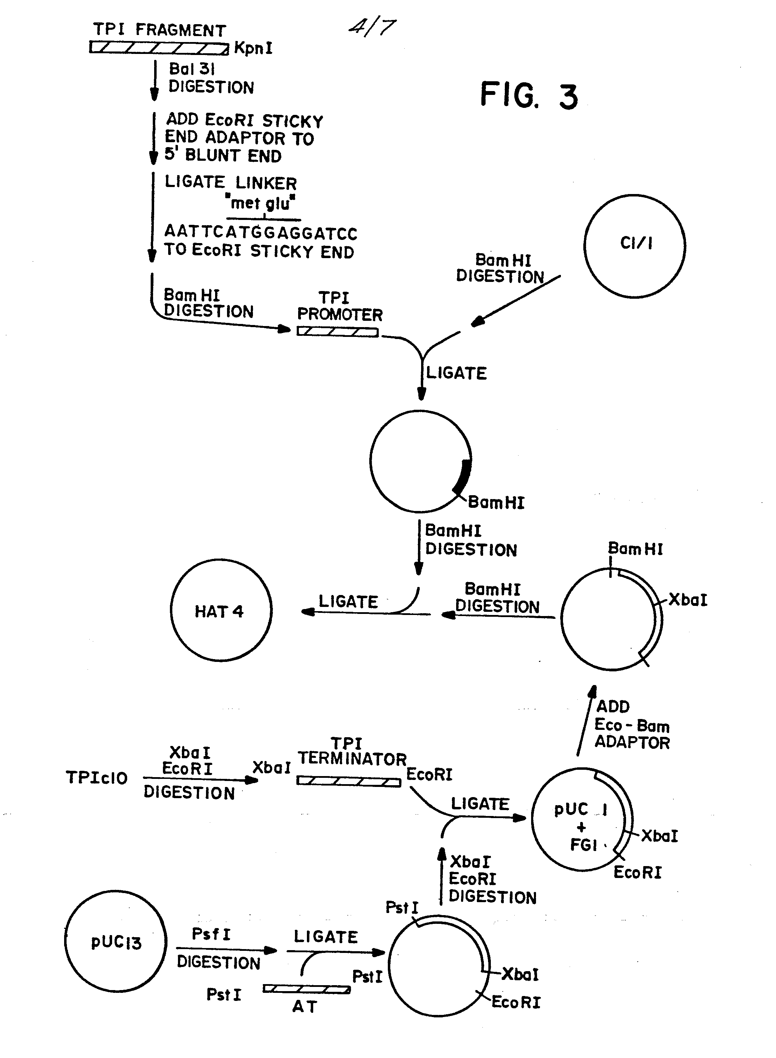

The gene for the predominant form of human alpha-1 antitrypsin is shown in FIG. 1. A DNA fragment comprising such gene which may be utilized may be derived from the plasmid HAT 4. The construction of HAT 4 is illustrated generally in FIG. 3.

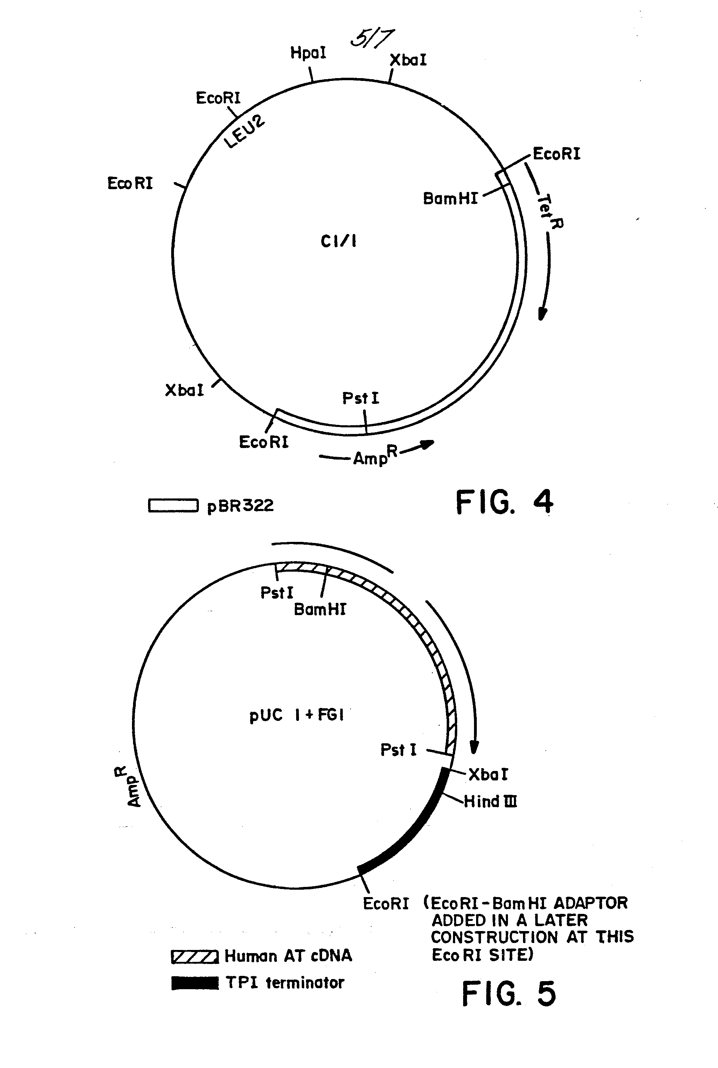

To prepare HAT 4, the intermediate vector Cl/1 is utilized. Referring to FIG. 3, the S. cerevisiae TPI promoter, described by Alber and Kawasaki, J.Molec.Appl. Genet., 1, 419-434 (1982) is digested with Bal31 exonuclease from the KpnI restriction site within the TPI coding region. The resultant blunt end is converted to a Eco RI sticky end by the addition of a 5' blunt end Eco RI adaptor. A synthetic oligonucleotide linker having the sequence AATTCATGGAGGATCC is ligated to the Eco RI sticky end, then digested with Bam HI. The resultant fragment comprising the TPI promoter may be inserted into the Bam HI site of the vector Cl/1. The restriction map of Cl/1 is shown in FIG. 4. The Cl/1 plasmid was constructed from plasmid pJDB248 (Beggs, Nature, 275, 104-109 (1978)) by removing the pMB9 sequences from pJDB248 by a partial digestion with Eco RI. Eco RI digested pBR322 was then inserted to give Cl/1. It will be noted that the linker sequence, in addition to providing Eco RI and Bam HI restriction sites, also supplies an ATG initiation codon and a GAG codon, which codes for glutamic acid, the first amino acid of naturally occurring human alpha-1 antitrypsin. Therefore, these codons are "in frame" when the rest of the alpha-1 antitrypsin structural gene is inserted at the Bam HI site.

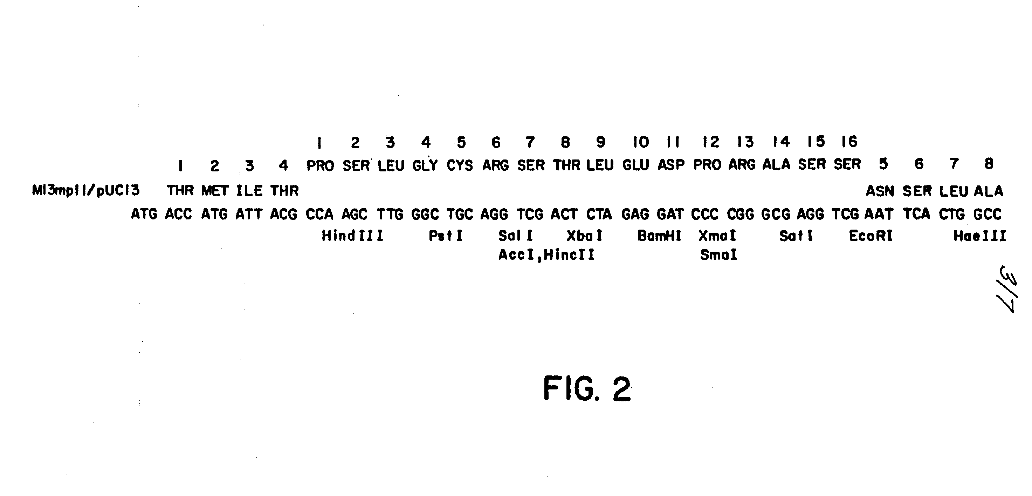

The gene coding for the predominant form of human alpha-1 antitrypsin (shown in FIG. 1) may be isolated from a human cDNA library using the baboon sequence (Kurachi et al., PNAS USA, 78, 6826-6830 (1981); and Chandra et al., Biochem.Biophys.Res.Com., 103, 751-758 (1981)) as a DNA hybridization probe. This gene may then be inserted as a Pstl fragment, as shown in FIG. 3 into vector pUC13 (prepared as described by Vieira et al., Gene, 19, 259-268 (1982) for vectors pUC8 and pUC9, but containing the multiple restriction site shown in FIG. 2 at start of the lacZ gene) at the single Pstl site in pUC13. In the resultant construct the antitrypsin gene was followed by an XbaI site and an Eco RI site in the multiple cloning sequence. Therefore, between these two sites was inserted the yeast TPI terminator as a 700 base pair XbaI-Eco RI fragment from pTPIC10. The plasmid pTPIC10 is described by Alber and Kawasaki, J.Molec.App.Gene., 1, 419-434 (1982). The resultant plasmid shown in FIG. 3 is pUCα-1 + FG1 which contains the human alpha-1 antitrypsin with a yeast transcription terminator. A detailed structure of pUCα-1 + FG1 is shown in FIG. 5.

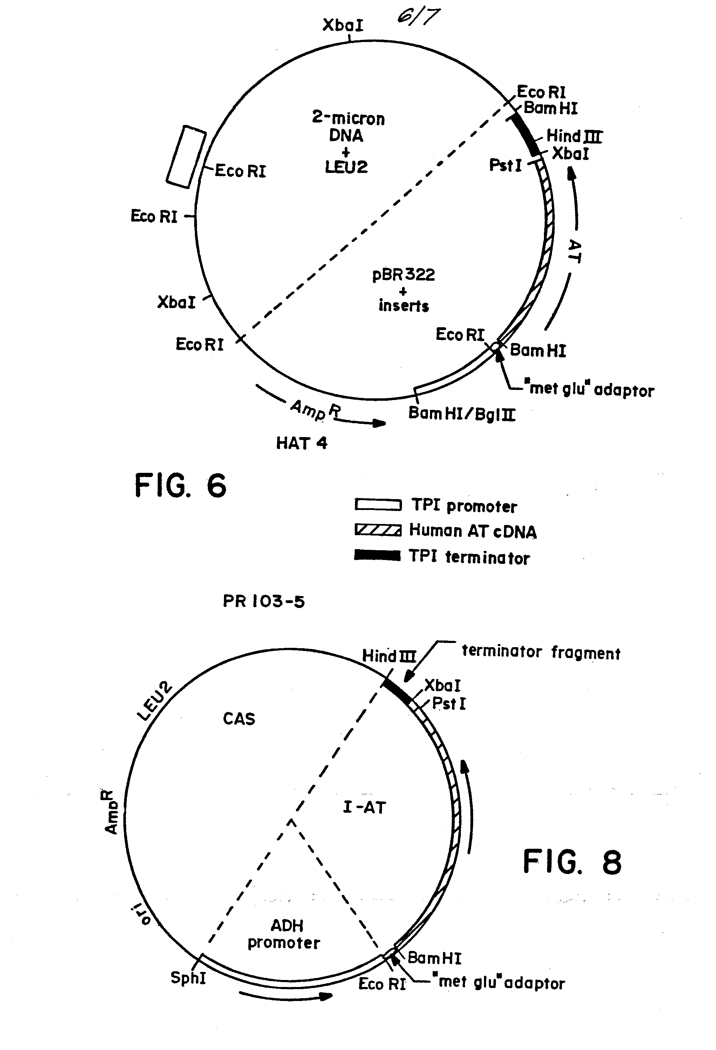

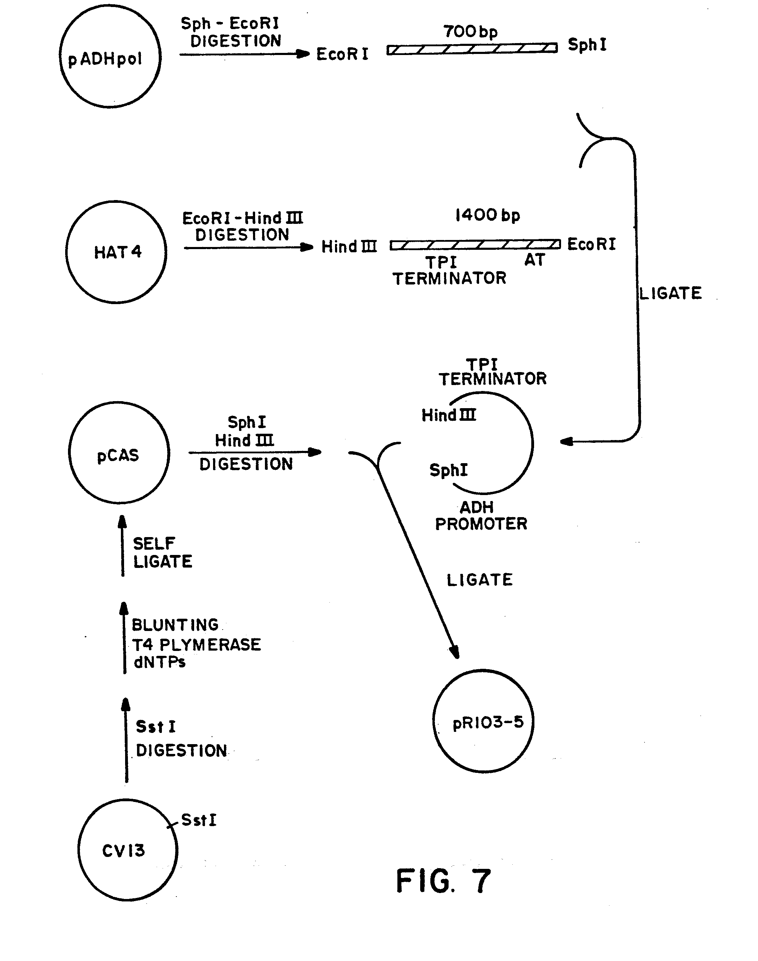

Referring to FIG. 3 an Eco RI, Bam HI synthetic DNA adaptor is then added to the Eco RI site of pUCα-1 + FG1 in order to create a Bam HI site on the 3' end of the yeast terminator. By using this adaptor the human alpha-1 antitrypsin yeast terminator sequence may be removed by cutting with Bam HI to liberate a fragment of approximately 2100 base pairs. This Bam HI fragment is then inserted into the modified C1/1 plasmid containing the TPI promoter with Bam HI adaptor. The resultant plasmid HAT 4 contains the TPI promoter, ATGGAG adaptor, human alpha-1 antitrypsin gene and TPI terminator. A detailed structure of HAT 4 is shown in FIG. 6. Referring to FIG. 7, HAT 4 was digested with Eco RI and Hind III and the approximately 1400 base pair Hind III-Eco RI fragment was isolated. This fragment was ligated to the Eco RI-Sph I 700 base pair fragment containing the S. pombe ADH promoter discussed above. The resultant Hind III-Sph I linear segment was then inserted into Sph I and Hind III digested shuttle vector pCAS. Plasmid pCAS is a derivative of CV13 (also known as YEp13; Broach et al., Gene, 8, 121-123 (1979)) in which the single Sst1 site has been destroyed by cutting with Sst1, blunting the ends with T4 DNA polymerase and dNTP's, and religating the linear molecule. The final construct shown in FIGURE 7 containing the S. pombe ADH promoter, alpha-1 antitrypsin coding sequence, and TPI terminator is known as pR103-5. A detailed structure of pR103-5 is shown in FIG. 8. This plasmid also contains the S. cerevisiae LEU2 gene which complements the leu1-32 mutation of S. pombe.

The plasmid pR103-5 may be used to transform S. pombe strains by conventional techniques. Growth of the transformed cells will result in expression of polypeptides exhibiting the protease inhibition activity of human alpha-1 antitrypsin. These proteins may be useful for treatment of genetic alpha-1 antitrypsin deficiency and other disease states related to inadequate levels of the alpha-1 antitrypsin. Thus, conditions such as emphysema and other lung disorders related to progressive digestion of lung sacs may be treated, such as, chronic obstructive pulmonary disease or adult respiratory distress syndrome. Non-genetically related emphysema may be also be treated, such as, emphysema resulting from heavy smoking. Conditions not necessarily confined to the lungs may also be treated, such as, cystic fibrosis and arthritis. For a review of alpha-1 antitrypsin in deficiency see Gadek, J. E., and R. Crystl, "Alpha-1 Antitrypsin Deficiency", The Metabolic Basis of Inherited Disease, J. B. Stanbury, J. B. Wyngaarden and D. S. Fredrickson, McGraw-Hill, New York, pages 1450-1467 (1982).

The proteins according to the present invention may be admixed with conventional pharmaceutical carriers. Preferably the proteins may be administered intraveneously or by inhalation. While effective dosages may vary according to the severity of the condition and weight of the subject, dosages in the range of 0.5 to 10.0 gm/wk of a protein introduced intraveneously may, in many cases, be effective. Lower dosages may be effective if the method of administration is by inhalation. Oral administration may also be effective provided the alpha-1 antitrypsin is protected in capsules or coded carriers from premature degradation in the digestive tract. The following examples set forth specific embodiments according to the present invention, but the invention is not intended to be limited thereto.

EXAMPLE 1

EXPRESSION OF PROTEINS HAVING ALPHA-1 ANTITRYPSIN ACTIVITY FROM S. POMBE.

The pR103-5 expression vector is used to transform S. pombe strain PR118-4 (h-, leu1-32), ATCC Deposit

No. 20680. The strain is obtained by crossing strain PR131 (h+, leu1-32), described by Kohli et al., Genetics, 87, 471-489 (1977), and strain PRP17 (h-, cdc10-129), described by Nurse et al., Molecular and General Genetics, 146, 167-168 (1976), and isolating clones which give consistent colony size. The transformed cells may be grown under selective conditions described by Russell et al., Nature, 301, 167-169 (1983). RNA analysis using Northern blots (Thomas, PNAS USA, 77, 5201-5205 (1980)) and a $^{32}$P-labeled Eco RI-Hind III alpha-1 antitrypsin-TPI terminator probe indicates that high level of transcription of alpha-1 antitrypsin cDNA is directed by the ADH promoter. Cell extracts from the log phase cultures grown under selective conditions are analyzed by ELISA assay and show 0.85%-0.95% of total soluble protein to be protein exhibiting alpha-1 antitrypsin activity.

## EXAMPLE 2

CHARACTERIZATION OF PROTEINS FROM S. POMBE EXHBITING ALPHA-1 ANTITRYPSIN ACTIVITY.

Alpha-1 antitrypsin proteins from the S. pombe transformants above in Example 1 are further characterized by a purification on amino-adsorption column and electrophoresis on a polyacrylamide/SDS gel. The column is prepared by covalently attaching affinity purified antibodies to human alpha-1 antitrypsin to CNBr-activated Sepharose according to the method of Cuatrecasas, J.Biochem., 245, 3059 (1970). Disrupted cells are extracted with phosphate buffered saline pH 7.2 containing 0.5m NaCl and applied to the column. The column is eluted with 3m NaSCN and the recovered material is analyzed by electrophoresis on a polyacrylamide gel in the presence of sodium dodecyl sulfate. The alpha-1 antitrypsin proteins which are produced in S. pombe have molecular weights in the range of 42-43 kdal. Naturally occurring human alpha-1 antitrypsin has a molecular weight of approximately 54 kdal., having a carbohydrate composition of approximately 16% by weight. See Hodges et al., J.Biochem., 254, 8208-8212 (1979). The proteins having alpha-1 antitrypsin activity produced by S. pombe will thus appear to be unglycosylated or substantially unglycosylated.


EXAMPLE 3


ELISA Assay for AT


To assay for AT, 200 microliters of goat anti-alpha-1-antitrypsin (10 micrograms/ml in 0.1M $Na_2CO_3$ pH 9.8) that has been affinity purified is placed in each well of a 96 well microtiter culture plate at 37°C for two to four hours. This solution is removed and 200 microliters of phosphate buffered saline (PBS) pH 7.2 containing 1% BSA and .05% Tween 20 is added to each well at 37°C for two hours.

Test samples, including standards, are then added to each well and AT, if present, binds to the specific antibody. The wells are rinsed with PBS after 15-30 minutes of incubation. 200 microliters of rabbit anti-alpha-1-AT is added to each well in the presence of 1% BSA in PBS. After 15 minutes incubation, the solution is removed and the wells are rinsed with PBS.

A sample of 200 microliters of goat anti rabbit IgG coupled to alkaline phosphatase in 1% BSA and PBS are added to each well and incubated for 15 minutes. After rinsing the wells, 200 microliters of a substrate solution was added which contains 30 milligrams of dinitrophenyl phosphate dissolved in 50 ml of 0.1 m diethanolamine pH 9.6. A yellow color is allowed to develop (15-60 minutes) and intensity is recorded at 405 nm on a Titertek Multiscan detector. The yellow color is proportional to the amount of AT in the test solution.

CLAIMS:

1. A method of producing a heterologous polypeptide in yeast Schizosaccharaomyces pombe comprising the step of growing a culture of said yeast transformed by a DNA vector, said vector comprising a segment coding for an S. pombe promotor, wherein expression of said polypeptide is under regulation of said promotor.

2. A method as claimed in claim 1, wherein said promotor comprises the alcohol dehydrogenase promotor.

3. A method as claimed in claim 1 or claim 2, wherein said polypeptide possesses the protease inhibition activity of mammalian alpha-1-antitrypsin.

4. A method as claimed in claim 3, wherein said polypeptide has the amino acid sequence of a form, e.g. the predominant form, of mammalian alpha-1-antitrypsin.

5. A method as claimed in claim 1, wherein said vector comprises plasmid pR103-5.

6. A method as claimed in any one of claims 1 to 5, wherein said polypeptide is substantially unglycosylated.

7. A method as claimed in any one of claims 1 to 6, wherein said polypeptide is substantially pure.

8. A DNA construct comprising a promotor of S. pombe and a gene for a heterologous protein positioned such that expression of said protein in S. pombe is under regulation of said promotor.

9. A construct as claimed in claim 8 and further defined by the specific feature of any one of claims 2 to 4.

10. A DNA construct as claimed in claim 8 comprising the DNA sequence of pR103-5.

11. A method of constructing a DNA vector capable in S. pombe of expressing a heterologous polypeptide, e.g. mammalian alpha-1-antitrypsin, comprising ligating DNA segments comprising respectively a structural gene encoding said polypeptide and a promotor indigenous to S. pombe.

12.  A transformant strain of S. pombe containing a DNA construct as defined in any one of claims 8 to 10.

13.  A method of forming a transformant strain of S. pombe having the capability of producing a heterologous polypeptide, e.g. a polypeptide having the protease inhibition activity of mammalian alpha-1-antitrypsin, which method comprises introducing a DNA transfer vector comprising a construct as defined in any one of claims 8 to 10 into S. pombe to transform the same thereby altering its genotype to encode in expressible format for said polypeptide.

14.  A therapeutic formulation for treating disease states related to inadequate amounts of alpha-1-antitrypsin in a mammal comprising a polypeptide which has been produced by a method as claimed in any one of claims 3 to 5 or in claim 6 or claim 7 when dependent directly or indirectly upon any one of claims 3 to 5 formulated for such treatment, e.g. for administration by inhalation, for oral administration or for intravenous administration.

15.  A method of producing a therapeutic formulation for treating disease states related to inadequate amounts of alpha-1-antitrypsin in a mammal comprising formulating for such treatment a polypeptide which has been produced by a method as claimed in any one of claims 3 to 5 or in claim 6 or claim 7 when dependent directly or indirectly upon any one of claims 3 to 5.

1/7

# FIG. I

```
                    -24              -20                                        -10
                    Met Pro Ser Ser Val Ser Trp Gly Ile Leu Leu Leu Ala-Gly Leu
TGG GAC AGT GAA TCG ACA ATG CCG TCT TCT GTC TCG TGG GGC ATC CTC CTG CTG GCA GGC CTG
30          40          50          60          70          80          90

                              -1  +1                                10
Cys Cys Leu Val Pro Val Ser Leu Ala Glu Asp Pro Gln Gly Asp Ala Ala Gln Lys Thr Asp
TGC TGC CTG GTC CCT GTC TCC CTG GCT GAG GAT CCC CAG GGA GAT GCT GCC CAG AAG ACA GAT
          100         110         120         130         140         150

                              20                                30
Thr Ser His His Asp Gln Asp His Pro Thr Phe Asn Lys Ile Thr Pro Asn Leu Ala Glu Phe
ACA TCC CAC CAT GAT CAG GAT CAC CCA ACC TTC AAC AAG ATC ACC CCC AAC CTG GCT GAG TTC
          160         170         180         190         200         210

                        40                                50
Ala Phe Ser Leu Tyr Arg Gln Leu Ala His Gln Ser Asn Ser Thr Asn Ile Phe Phe Ser Pro
GCC TTC AGC CTA TAC CGC CAG CTG GCA CAC CAG TCC AAC AGC ACC AAT ATC TTC TTC TCC CCA
220         230         240         250         260         270         280

                  60                                70
Val Ser Ile Ala Thr Ala Phe Ala Met Leu Ser Leu Gly Thr Lys Ala Asp Thr His Asp Glu
GTG AGC ATC GCT ACA GCC TTT GCA ATG CTC TCC CTG GGG ACC AAG GCT GAC ACT CAC GAT GAA
            290         300         310         320         330         340

                  80                                90
Ile Leu Glu Gly Leu Asn Phe Asn Leu Thr Glu Ile Pro Glu Ala Gln Ile His Glu Gly Phe
ATC CTG GAG GGC CTG AAT TTC AAC CTC ACG GAG ATT CCG GAG GCT CAG ATC CAT GAA GGC TTC
            350         360         370         380         390         400

            100                               110
Gln Glu Leu Leu Arg Thr Leu Asn Gln Pro Asp Ser Gln Leu Gln Leu Thr Thr Gly Asn Gly
CAG GAA CTC CTC CGT ACC CTC AAC CAG CCA GAC AGC CAG CTC CAG CTG ACC ACC GGC AAT GGC
      410         420         430         440         450         460         470

            120                               130
Leu Phe Leu Ser Glu Gly Leu Lys Leu Val Asp Lys Phe Leu Glu Asp Val Lys Lys Leu Tyr
CTG TTC CTC AGC GAG GGC CTG AAG CTA GTG GAT AAG TTT TTG GAG GAT GTT AAA AAG TTG TAC
            480         490         500         510         520         530

        140                               150
His Ser Glu Ala Phe Thr Val Asn Phe Gly Asp Thr Glu Glu Ala Lys Lys Gln Ile Asn Asp
CAC TCA GAA GCC TTC ACT GTC AAC TTC GGG GAC ACC GAA GAG GCC AAG AAA CAG ATC AAC GAT
        540         550         560         570         580         590

160                                170                               180
Tyr Val Glu Lys Gly Thr Gln Gly Lys Ile Val Asp Leu Val Lys Glu Leu Asp Arg Asp Thr
TAC GTG GAG AAG GGT ACT CAA GGG AAA ATT GTG GAT TTG GTC AAG GAG CTT GAC AGA GAC ACA
    600         610         620         630         640         650

                          190                               200
Val-Phe Ala Leu Val Asn Tyr Ile Phe Phe Lys Gly Lys Trp Glu Arg Pro Phe Glu Val Lys
GTT TTT GCT CTG GTG AAT TAC ATC TTC TTT AAA GGC AAA TGG GAG AGA CCC TTT GAA GTC AAG
660         670         680         690         700         710         720

                          210                               220.
Asp Thr Glu Glu Glu Asp Phe His Val Asp Gln Val Thr Thr Val Lys Val Pro Met Met Lys
GAC ACC GAG GAA GAG GAC TTC CAC GTG GAC CAG GTG ACC ACC GTG AAG GTG CCT ATG ATG AAG
    730         740         750         760         770         780

                          230                               240
Arg Leu Gly Met Phe Asn Ile Gln His Cys Lys Lys Leu Ser Ser Trp Val Leu Leu Met Lys
CGT TTA GGC ATG TTT AAC ATC CAG CAC TGT AAG AAG CTG TCC AGC TGG GTG CTG CTG ATG AAA
    790         800         810         820         830         840
```

# FIG. 1 - page 2

```
              250                                    260
Tyr Leu Gly Asn Ala Thr Ala Ile Phe Phe Leu Pro Asp Glu Gly Lys Leu Gln His Leu Glu
TAC CTG GGC AAT GCC ACC GCC ATC TTC TTC CTG CCT GAT GAG GGG AAA CTA CAG CAC CTG GAA
850          860         870         880         890         900         910


              270                                    280
Asn Glu Leu Thr His Asp Ile Ile Thr Lys Phe Leu Glu Asn Glu Asp Arg Arg Ser Ala Ser
AAT GAA CTC ACC CAC GAT ATC ATC ACC AAG TTC CTG GAA AAT GAA GAC AGA AGG TCT GCC AGC
             920         930         940         950         960         970


              290                                    300
Leu His Leu Pro Lys Leu Ser Ile Thr Gly Thr Tyr Asp Leu Lys Ser Val Leu Gly Gln Leu
TTA CAT TTA CCC AAA CTG TCC ATT ACT GGA ACC TAT GAT CTG AAG AGC GTC CTG GGT CAA CTG
          980         990         1000        1010        1020        1030


           310                                    320
Gly Ile Thr Lys Val Phe Ser Asn Gly Ala Asp Leu Ser Gly Val Thr Glu Glu Ala Pro Leu
GGC ATC ACT AAG GTC TTC AGC AAT GGG GCT GAC CTC TCC GGG GTC ACA GAG GAG GCA CCC CTG
   1040        1050        1060        1070        1080        1090        1100


        330                                    340              350
Lys Leu Ser Lys Ala Val His Lys Ala Val Leu Thr Ile Asp Glu Lys Gly Thr Glu Ala Ala
AAG CTC TCC AAG GCC GTG CAT AAG GCT GTG CTG ACC ATC GAC GAG AAA GGG ACT GAA GCT GCT
            1110        1120        1130        1140        1150        1160


                                          360              370
Gly Ala Met Phe Leu Glu Ala Ile Pro Met Ser Ile Pro Pro Glu Val Lys Phe Asn Lys Pro
GGG GCC ATG TTT TTA GAG GCC ATA CCC ATG TCT ATC CCC CCC GAG GTC AAG TTC AAC AAA CCC
   1170        1180        1190        1200        1210        1220


                                       380                       390
Phe Val Phe Leu Met Ile Glu Gln Asn Thr Lys Ser Pro Leu Phe Met Gly Lys Val Val Asn
TTT GTC TTC TTA ATG ATT GAA CAA AAT ACC AAG TCT CCC CTC TTC ATG GGA AAA GTG GTG AAT
   1230        1240        1250        1260        1270        1280


        394
Pro Thr Gln Lys STOP
CCC ACC CAA AAA TAA CTG CCT CTC GCT CCT CAA CCC CTC CCC TCC ATC CCT GGC CCC CTC CCT
1290        1300        1310        1320        1330        1340        1350



GGA TGA CAT TAA AGA AGG GTT GAG CTG
     1360         1370



G AAAAAAAAAAAAAAAA CCCCCCCCCCCCCCCCCCCCCCCCCCCCCCCCCCCCCCCCCC 3'
1380      1390      1400      1410      1420      1430
```

|  | 1 | 2 | 3 | 4 | 5 | 6 | 7 | 8 | 9 | 10 | 11 | 12 | 13 | 14 | 15 | 16 |
|---|---|---|---|---|---|---|---|---|---|----|----|----|----|----|----|----|

1 2 3 4 PRO SER LEU GLY CYS ARG SER THR LEU GLU ASP PRO ARG ALA SER SER 5 6 7 8

M13mp11/pUC13  THR MET ILE THR                                                                 ASN SER LEU ALA

ATG ACC ATG ATT ACG CCA AGC TTG GGC TGC AGG TCG ACT CTA GAG GAT CCC CGG GCG AGG TCG AAT TCA CTG GCC

HindIII        PstI       SalI      XbaI      BamHI   XmaI       SatI      EcoRI        HaeIII

AccI,HincII                  SmaI

# FIG. 2

FIG. 3

5/7

FIG. 4

☐ pBR322

▨ Human AT cDNA

■ TPI terminator

FIG. 5

0139383

FIG. 6

TPI promoter
Human AT cDNA
TPI terminator

FIG. 8

FIG. 7

0139383

**European Patent Office**

**EUROPEAN SEARCH REPORT**

Application number

EP 84305571.6

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl.4) |
| A | NATURE, vol. 290, no. 5802, March 12, 1981 (New York, London) <br><br> D. BEACH et al. "High-frequency transformation of the fission yeast Schizosaccharomyces pombe" pages 140-142 <br><br> * Totality * | 1,8, 11,13 | C 12 N 15/00 <br> C 12 P 21/00 <br> C 12 N 9/99 <br> A 61 K 37/02 <br> //C 12 R 1/85 |
| A | MOLECULAR & GENERAL GENETICS, vol. 182, no. 3, 1981 (Springer Verlag, Berlin, Heidelberg) <br><br> M. YAMAMOTO et al. "Cloning of a Gene from the Fission Yeast S. pompe which Complements E. coli pyr B, the Gene for Aspartate Trans-carbamylase" pages 426-429 <br><br> * Totality * | 1,8, 11,13 | |
| | | | **TECHNICAL FIELDS SEARCHED (Int. Cl.4)** |
| D,A | PROCEEDINGS OF THE NATIONAL ACADE-MY OF SCIENCES OF THE UNITED STATES OF AMERICA, vol. 78, no. 11, November 1981 (Baltimore, USA) <br><br> K. KURACHI et al. "Cloning and se-quence of cDNA coding for $\alpha_1$-anti-trypsin" pages 6826-6830 <br><br> * Abstract * | 1,3,4, 11,13 | C 12 N <br> C 12 P <br> A 61 K <br> C 07 H |

The present search report has been drawn up for all claims

| Place of search | Date of completion of the search | Examiner |
|---|---|---|
| VIENNA | 23-11-1984 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

0139383

**European Patent Office**

**EUROPEAN SEARCH REPORT**

Application number

| DOCUMENTS CONSIDERED TO BE RELEVANT | | | EP 84305571.6 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | BIOCHEMICAL AND BIOPHYSICAL RESEARCH COMMUNICATIONS, vol. 103, no. 2, November 30, 1981 (New York, London, Toronto, Sydney, San Francisco, Tokyo)<br><br>T. CHANDRA et al. "Induction of $\alpha_1$-Antitrypsin mRna and cloning of its cDNA"<br>pages 751-758<br><br>   * Summary * <br><br>     ---- | 1,3,4, 11,13 | |
| | | | **TECHNICAL FIELDS SEARCHED (Int. Cl.4)** |

| The present search report has been drawn up for all claims | | |
|---|---|---|
| Place of search | Date of completion of the search | Examiner |
| VIENNA | 23-11-1984 | WOLF |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503 03 82